# EUROPÄISCHE PATENTSCHRIFT

(11) **EP 2 897 556 B1**
(45) Veröffentlichungstag und Bekanntmachung des Hinweises auf die Patenterteilung: **09.08.2017**
(21) Anmeldenummer: 13765721.9
(22) Anmeldetag: 19.09.2013
(51) Int. Cl.: A61F 2/24

(54) **HERZKLAPPENPROTHESE**
HEART VALVE PROSTHESIS
PROTHÈSE VALVULAIRE CARDIAQUE

(30) Priorität: 19.09.2012 DE 102012216742
(43) Veröffentlichungstag der Anmeldung: 29.07.2015
(73) Patentinhaber: Sievers, Hans-Hinrich, 24119 Kronshagen (DE)
(72) Erfinder: Sievers, Hans-Hinrich, 24119 Kronshagen (DE)
(74) Vertreter: Patentanwälte Vollmann & Hemmer
(86) Internationale Anmeldenummer: PCT/EP2013/069486
(87) Internationale Veröffentlichungsnummer: WO 2014/044762

(56) Entgegenhaltungen:
- DE-C1- 19 633 346
- US-A- 4 655 772
- US-B1- 6 228 112

## Beschreibung

Die Erfindung betrifft eine Herzklappenprothese mit den im Oberbegriff des Anspruchs 1 angegebenen Merkmalen.

Aus EP 1 703 865 B1 ist eine Herzklappenprothese bekannt, welche einen Ringkörper aufweist, welcher an seinem Außenumfang mit dem Gewebe vernäht wird und an seinem Innenumfang mehrere bewegliche Klappenelemente trägt. Dabei sind die Klappenelemente an ihren Seitenkanten gelenkig gelagert. Die Gelenke sind dabei ringseitig an Vorsprüngen ausgebildet, welche sich in das Innere des Ringkörpers hinein erstrecken. Nachteilig bei dieser Konstruktion ist, dass im Bereich dieser Gelenke nicht von Blut durchströmte Bereiche vorhanden sind, an welchen sich Ablagerungen bilden können.

Aus US 4 655 772 A sowie US 6 228 112 B1 sind mechanische Herzklappenprothesen bekannt, bei welchen die Klappenelemente Durchbrechungen aufweisen, in welche Arme zum Halten der Klappenelemente eingreifen. Auch diese Ausgestaltung hat den Nachteil, dass nicht von Blut durchströmte Toträume vorhanden sind, in welchen sich Ablagerungen bilden können.

Im Hinblick auf diese Problematik ist es Aufgabe der Erfindung, eine verbesserte Herzklappenprothese bereitzustellen, welche ein verringertes Risiko von unerwünschten Ablagerungen aufweist. Diese Aufgabe wird gelöst durch eine Herzklappenprothese mit den in Anspruch 1 angegebenen Merkmalen. Bevorzugte Ausführungsformen ergeben sich aus den Unteransprüchen sowie der nachfolgenden Beschreibung und den beigefügten Figuren.

Die erfindungsgemäße Herzklappenprothese ist eine mechanische Herzklappenprothese und weist einen Ringkörper auf, welcher vorzugsweise an seinem Außenumfang einen Nahtring zum Vernähen des Ringkörpers mit dem umgebenden Gewebe aufweist. In dem Ringkörper, welcher eine feste Struktur bildet, sind mehrere Klappenelemente beweglich gelagert. Dazu sind die Klappenelemente mit dem Ringkörper über Gelenke beweglich verbunden. Die Gelenke ermöglichen eine Schwenkbewegung der Klappenelemente zwischen einer geschlossenen Stellung, in der die mehreren Klappenelemente vorzugsweise aneinander anliegen, und einer geöffneten Stellung, in welcher die Klappenelemente im Wesentlichen in Strömungsrichtung des Blutes gerichtet sind und den Innenraum des Ringkörpers freigeben. Die Klappenelemente sind bevorzugt an den Gelenken so angeordnet, dass sie jeweils um eine Schwenkachse schwenkbar sind, welche sich normal zur Strömungsrichtung und bevorzugt sehnenförmig in dem Ringkörper erstreckt. Dabei erstrecken sich die Schwenkachsen bevorzugt jeweils so, dass sie sich quer, d. h. insbesondere normal zu einer Symmetrieachse jedes Klappenelementes erstrecken. Die Klappenelemente sind vorzugsweise symmetrisch zu einer sich radial zum Mittelpunkt der Herzklappenprothese erstreckenden Symmetrieachse ausgebildet. Zu dieser Symmetrieachse erstreckt sich die Schwenkachse des Klappenelementes bevorzugt normal. Bevorzugt sind drei Klappenelemente vorgesehen, wobei dann weiter bevorzugt die drei Schwenkachsen der drei Klappenelemente ein gleichschenkliges Dreieck aufspannen, wobei die Spitzen des Dreiecks jeweils auf der Symmetrieachse eines entgegengesetzt gelegenen Klappenelementes gelegen sind. Die Symmetrieachsen der drei Klappenelemente schneiden sich im Mittelpunkt der Herzklappenprothese. Bei dieser Anordnung der Schwenkachsen schwenken die Klappenelemente ausgehend von diesem Mittelpunkt in radialer Richtung auf. D. h. die am Mittelpunkt gelegene Spitze jedes Klappenelementes verschwenkt auf einer Kreisbahn um die Schwenkachse, welche in einer Ebene gelegen ist, welche die Mittelachse der Herzklappenprothese schneidet und sich parallel zu dieser erstreckt. Gleichzeitig ist die Symmetrieachse jedes Klappenelementes in dieser Schwenkebene gelegen.

Erfindungsgemäß ist vorgesehen, dass die Gelenke an den Klappenelementen an deren ventrikulärer Oberfläche angreifen. Die ventrikuläre Oberfläche ist dabei diejenige Klappenoberfläche, welche von dem Blut in dessen Strömungsrichtung angeströmt wird, das heißt die in Strömungsrichtung des Blutes stromaufwärts gelegene Oberfläche, welche der Herzkammer zugewandt ist. Die Anordnung der Gelenke ausschließlich an der ventrikulären bzw. systolischen, d. h. vom Blut zum Öffnen der Herzklappe direkt angeströmten Seite hat den Vorteil, dass die Gelenkbereiche vollständig vom Blut über- bzw. durchströmt werden, so dass sich im Bereich der Gelenke keine Anlagerungen bilden können. Die aortale, der Herzkammer abgewandte Oberfläche der Klappenelemente ist hingegen frei von Bestandteilen der Gelenke.

Die Gelenke greifen an den Klappenelementen bevorzugt jeweils beabstandet von deren Seiten- bzw. Umfangskante an der ventrikulären Oberfläche an. Dies hat den Vorteil, dass die Kanten der Klappenelemente frei von Gelenken sind, so dass auch die Kanten im geöffneten Zustand der Herzklappe frei vom Blut überströmt werden können und sich in diesem Bereich keine Anlagerungen bilden können. Insbesondere weist jedes Klappenelement bei dieser Ausgestaltung vorzugsweise Gelenke auf, welche unabhängig von den Gelenken der übrigen Klappenelemente sind. Dies ermöglicht es, die Gelenke und die die Gelenke tragenden Strukturen insgesamt schlanker auszubilden, so dass es weniger Hinterschneidungen und Totbereiche gibt, welche nicht vom Blut durchströmt werden und in welchen sich Anlagerungen ansammeln könnten. Insgesamt wird so erreicht, dass vorzugsweise alle Bereiche der Herzklappenprothese im geöffneten Zustand der Klappenelemente direkt vom Blut durchströmt werden, so dass sich keine Anlagerungen bilden können.

Die Klappenelemente sind jeweils weiter bevorzugt mit zwei Gelenken an dem Ringkörper befestigt. Die Schwenkachse des Klappenelementes erstreckt sich dabei durch die beiden Gelenke. Durch die beiden Gelenke wird sichergestellt, dass die Klappenelemente definiert nur um diese eine Schwenkachse verschwenken. Vorzugsweise sind die beiden Gelenke symmetrisch an dem Klappenelement angeordnet, d. h. symmetrisch an beiden Seiten einer Mittel- bzw. Symmetrieachse des Klappenelementes gelegen. Die Symmetrieachse erstreckt sich vorzugsweise in radialer Richtung zum Mittelpunkt bzw. zur Spitze des Klappenelementes, welche im geschlossenen Zustand am Mittelpunkt der Herzklappenprothese gelegen ist. Ferner sind bevorzugt beide Gelenke gleich weit von dem Ringkörper beabstandet. Die Verwendung von zwei Gelenken pro Klappenelement ist insbesondere dann von Vorteil, wenn die Gelenke als Kugelgelenke ausgestaltet sind, sodass die beiden Gelenke gemeinsam die definierte Schwenkachse festlegen können. Es ist jedoch auch denkbar, lediglich ein Gelenk pro Klappenelement zu verwenden, wenn dieses bevorzugt eine Schwenkbewegung nur um eine definierte Schwenkachse zulässt.

Die Gelenke weisen jeweils einen an dem Ringkörper angebrachten Gelenkkopf auf, welcher in eine an der ventrikulären Oberfläche des Klappenelementes ausgebildete Gelenkpfanne eingreift. Diese Gelenke sind weiter bevorzugt als Kugelgelenke ausgebildet, d. h. der Gelenkkopf weist die Form eines Kugelabschnittes auf, und die Gelenkpfanne weist eine korrespondierende konkave Kugelform bzw. Form eines Kugelabschnittes auf. Der Gelenkkopf und die Gelenkpfanne sind bevorzugt so dimensioniert, dass der Gelenkkopf möglichst spielfrei in die Gelenkpfanne eingreift, die Gelenkpfanne sich jedoch zu dem Gelenkkopf frei drehen bzw. bewegen kann, um die Beweglichkeit des Klappenelementes zu gewährleisten. Es findet eine Schmierung durch das Blut statt. Der Gelenkspalt selber ist so klein, dass sich dort keine Ablagerungen anlagern können. Durch die Anordnung von zwei Gelenken pro Klappenelement wird trotz der Ausgestaltung der einzelnen Gelenke als Kugelgelenk sichergestellt, dass eine Schwenkbewegung nur um eine definierte Schwenkachse, welche sich durch beide Gelenke erstreckt, möglich ist. Eine Ausgestaltung als Kugelgelenk vereinfacht jedoch die Montage und die Fertigung und garantiert eine dauerhaft leichte Beweglichkeit der Klappenelemente an den Gelenken.

Vorzugsweise sind die Gelenke ringkörperseitig an Vorsprüngen ausgebildet, welche sich in den Innenraum des Ringkörpers hinein erstrecken. Dadurch wird erreicht, dass die Gelenke selber im Innenraum des Ringkörpers gelegen sind, in welchem eine starke Blutströmung herrscht, so werden die Gelenkbereiche gut von Blut durchströmt und die Bildung von Ablagerungen verhindert. Die Vorsprünge weisen bevorzugt eine solche Länge auf, dass das an ihnen gelegene Gelenk auf einer radialen Linie, welche vom Mittelpunkt der Herzklappenprothese zum Ringkörper durch das Gelenk hindurch verläuft, vom Ringkörper um ein Maß beabstandet ist, welches mehr als ein Drittel, vorzugsweise mehr als die Hälfte des Abstandes zwischen Ringkörper und Mittelpunkt beträgt. Die Vorsprünge selber können so schmal dimensioniert werden, dass sie vollständig vom Blut umströmt werden, so dass sich in Strömungsrichtung des Blutes gesehen an der Rückseite der Vorsprünge ebenfalls keine Ablagerungen bilden können. Die Vorsprünge sind somit bevorzugt als schmale Stege bzw. Arme ausgebildet. In dem Fall, dass zwei Gelenke pro Klappenelement vorgesehen sind, sind bevorzugt ebenfalls zwei Vorsprünge pro Klappenelement an dem Ringkörper ausgebildet, sodass jedes Gelenk an einem einzelnen Vorsprung gelegen ist. Bevorzugt sind in dem Fall, dass zwei Gelenke mit zwei Vorsprüngen pro Klappenelement vorgesehen sind, diese symmetrisch zueinander bezüglich einer Symmetrieachse des Klappenelementes angeordnet. D. h. die Gelenke und Vorsprünge sind gleich und insbesondere spiegelsymmetrisch zueinander ausgebildet. Es ist jedoch auch denkbar, mehrere Gelenke an einem gemeinsamen Vorsprung anzuordnen. Für den Fall, dass nur ein Gelenk pro Klappenelement vorgesehen ist, wäre entsprechend bevorzugt auch nur ein Vorsprung vorzusehen, an welchem dieses Gelenk angeordnet ist.

Gemäß einer bevorzugten Ausführungsform der Erfindung erstrecken sich die Vorsprünge, an denen die Gelenke gelegen sind, nicht in einer Ebene normal bzw. senkrecht zur Längsachse der Herzklappenprothese, wobei die Längsachse der Strömungsrichtung entspricht. Bevorzugt erstrecken sich die Vorsprünge vielmehr in einem spitzen Winkel zu dieser Längsachse, d. h. in einem Winkel < 90°. Dadurch wird erreicht, dass sich diese Vorsprünge in geschlossenem Zustand der Herzklappenprothese ebenfalls gewinkelt zu der von den Klappenelementen aufgespannten Ebene erstrecken. So kann gerade in der Nähe des Ringkörpers ein größerer Abstand zwischen den Vorsprüngen und den Klappenelementen erreicht werden, so dass sich in diesem Bereich keine Ablagerungen zwischen den Vorsprüngen und den Klappenelementen bilden können. Die Vorsprünge kommen besonders bevorzugt ausschließlich mit den Gelenken mit den Klappenelementen in Berührung.

Die Vorsprünge erstrecken sich bevorzugt im Wesentlichen radial von dem Ringkörper in dessen Innenraum hinein, d. h. in einer Richtung quer zur Strömungsrichtung des Blutes. Vorzugsweise haben die Vorsprünge einen stromlinienförmigen Querschnitt, welcher die Umströmung des Blutes begünstigt. Dabei ist die Querschnittsform so gebildet, dass sich an der in Strömungsrichtung rückseitigen Fläche vorzugsweise kein Totbereich bildet, in dem keine Blutströmung herrscht. Auf diese Weise werden Ablagerungen an den Vorsprüngen verhindert.

Vorzugsweise weist die erfindungsgemäße Herzklappenprothese mindestens zwei Klappenelemente auf. Bei einer besonders bevorzugten Ausführungsform weist die Herzklappenprothese drei Klappenelemente auf, welche beweglich in dem Ringkörper angeordnet sind. Jedes der Klappenelemente ist dabei vorzugsweise über zwei Gelenke, welche an der ventrikulären Oberfläche des jeweiligen Klappenelementes angreifen, beweglich in dem Ringkörper gelagert. Die beiden Gelenke jedes Klappenelementes sind in Richtung der Schwenkachse voneinander beabstandet, so dass eine Drehung bzw. Bewegung quer zur Schwenkachse verhindert wird. Dabei sind die beiden Gelenke bevorzugt, wie oben beschrieben, symmetrisch, insbesondere spiegelsymmetrisch zueinander angeordnet. Bei der Anordnung von drei Klappenelementen sind bevorzugt insgesamt sechs Gelenke vorgesehen. Jedes Gelenk ist vorzugsweise an einem einzelnen Steg bzw. Vorsprung ausgebildet, welcher von dem Ringkörper radial nach innen in den Innenraum des Ringkörpers vorsteht. So wird einerseits eine stabile Lagerung geschaffen und darüber hinaus sichergestellt, dass sämtliche Bereiche der Herzklappenprothese von Blut durchströmt werden, um Ablagerungen zu verhindern.

Die Klappenelemente sind weiter bevorzugt derart ausgestaltet und mit ihren Schwenkachsen derart in dem Ringkörper angeordnet, dass sich die Klappenelemente bei Verschwenken in die geöffnete Stellung nicht über den Außenumfang des Ringkörpers hinaus erstrecken, sondern stets im Innenumfang des Ringkörpers bzw. im Inneren einer Projektion des Ringkörpers entlang der Längsachse der Herzklappenprothese verbleiben. So wird sichergestellt, dass das Aufschwenken in die geöffnete Stellung nicht durch umgebendes Gewebe behindert werden kann.

Die Klappenelemente sind besonders bevorzugt aus Kohlenstoff gefertigt bzw. als Kohlenstoffstruktur ausgebildet. Ein solches Material ist sehr resistent gegen Ablagerungen an der Oberfläche und weist darüber hinaus eine hohe Verschleißfestigkeit auf.

Der Ringkörper ist bevorzugt aus Metall, beispielsweise Titan gefertigt. Dabei kann der Ringkörper aus mehreren Teilen zusammengesetzt sein oder aber besonders bevorzugt einstückig ausgebildet sein. Eine einstückige Ausgestaltung vereinfacht die Fertigung und sorgt für eine stabile Struktur des Ringkörpers. Eine geteilte bzw. mehrteilige Ausbildung des Ringkörpers hingegen kann für die Montage der Klappenelemente von Vorteil sein.

Nachfolgend wird die Erfindung beispielhaft anhand der beigefügten Figuren beschrieben. In diesen zeigt:
- Fig. 1: eine perspektivische Gesamtansicht einer erfindungsgemäßen Herzklappenprothese im geschlossenen Zustand,
- Fig. 2: eine perspektivische Gesamtansicht einer erfindungsgemäßen Herzklappenprothese gemäß Fig. 1 im geöffneten Zustand,
- Fig. 3: eine Draufsicht auf die Herzklappenprothese gemäß Fig. 2 im geöffneten Zustand,
- Fig. 4: eine Draufsicht auf den Ringkörper ohne Klappenelemente,
- Fig. 5: eine vergrößerte Ansicht eines einzelnen Klappenelementes, und
- Fig. 6: eine Schnittansicht des Klappenelementes gemäß Fig. 5 entlang der Linie VI - VI in Fig. 5.

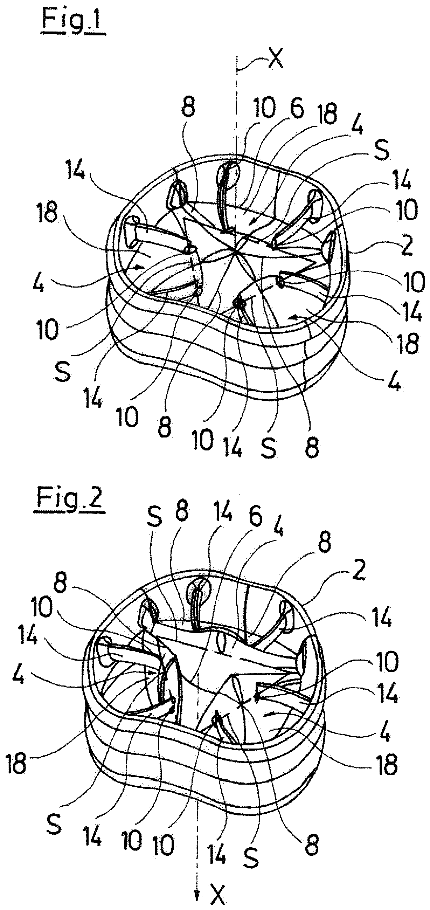

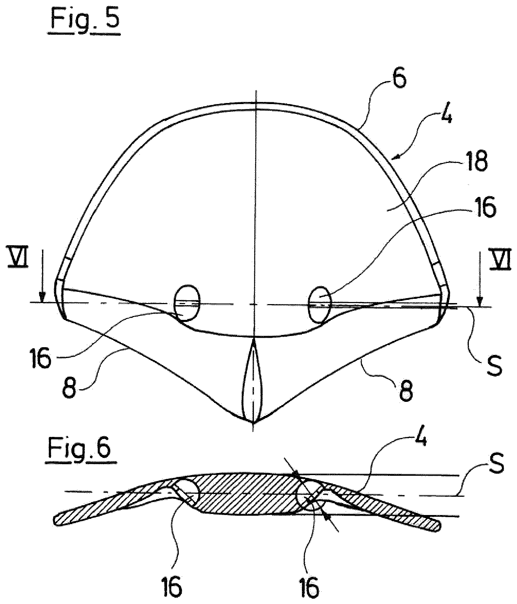

Die in den Figuren gezeigte mechanische Herzklappenprothese weist einen Ringkörper 2 mit drei darin angeordneten Klappenelementen 4 auf. Die Klappenelemente 4 sind im freien Innenraum des Ringkörpers 2 angeordnet und gelenkig an dem Ringkörper 4 gelagert, so dass sie zwischen der in Fig. 1 gezeigten geschlossenen Position und der in Fig. 2 gezeigten geöffneten Position um die Schwenkachsen S verschwenken können. In dem in Fig. 1 gezeigten geschlossenen Zustand liegen die Klappenelemente 4, 6 (siehe Fig. 5) am Innenumfang des Ringkörpers 2 und mit ihren beiden gewinkelt zueinander verlaufenden inneren Seitenkanten 8 aneinander an, so dass sie den freien Innenraum des Ringkörpers 2 verschließen und abdichten. Im geöffneten Zustand erstrecken sich die Klappenelemente 2 im Wesentlichen in der Strömungsrichtung X des Blutes, so dass sie den Innenraum des Ringkörpers 2 freigeben.

Die einzelnen Klappenelemente 4 sind jeweils an zwei Gelenken 10 gelenkig an dem Ringkörper 2 gelagert. Die Gelenke 10 weisen ringseitig einen Kugelkopf 12 auf, welcher am Ende eines Steges bzw. Vorsprunges 14 angeordnet ist. Die Vorsprünge 14 sind einstückig mit dem Ringkörper 2 ausgebildet und erstrecken sich von diesem im Wesentlichen in radialer Richtung nach innen in den Innenraum des Ringkörpers 2 hinein, so dass die an den freien Enden der Vorsprünge 14 ausgebildeten Kugelköpfe 12 beabstandet vom Innenumfang des Ringkörpers 2 gelegen sind. Vorzugsweise beträgt der radiale Abstand der Kugelköpfe 12 vom Innenumfang des Ringes mehr als ein Drittel des Radius des Ringkörpers 2 oder einer radialen Linie vom Mittelpunkt X durch den Kugelkopf 12 zum Ringkörper, vorzugsweise liegen die Kugelköpfe 4 in etwa am halben Radius.

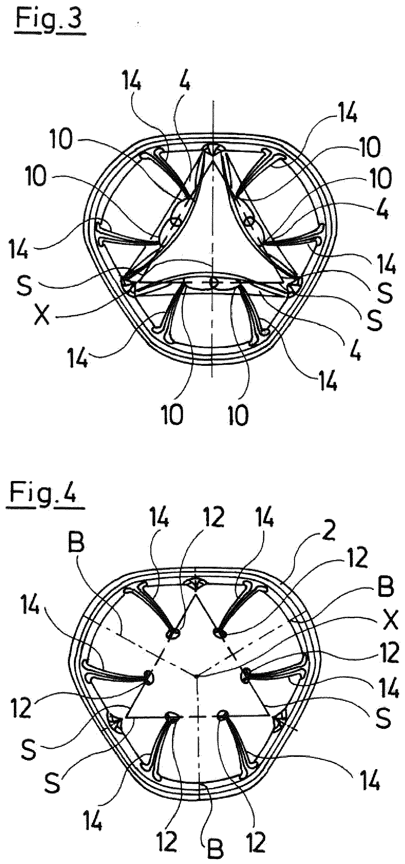

Die zu jedem Klappenelement 4 gehörenden zwei Vorsprünge 14 und deren Kugelköpfe 12 sind in Richtung der Schwenkachse S voneinander beabstandet. Dabei sind die Klappenelemente 4 und ihre zwei zugehörigen Vorsprünge 14 jeweils symmetrisch zu den Symmetrieachsen B ausgebildet. Das bedeutet, die zwei Vorsprünge 14 mit den Kugelköpfen 12 jedes Klappenelementes 4 sind spiegelsymmetrisch zu einer Symmetrieachse B angeordnet. Die drei Symmetrieachsen B der drei Klappenelemente 4 schneiden sich in dem Mittelpunkt bzw. der Längsachse X, welche der Strömungsrichtung entspricht (s. Fig. 4). Die Schwenkachse S jedes Klappenelementes 4 erstreckt sich durch die zugehörigen beiden Kugelköpfe 12, d. h. durch die beiden Gelenke 10. Die Kugelköpfe 12 greifen an den Klappenelementen 4 in Gelenkpfannen 16 ein, welche wie in Fig. 6 gezeigt, ebenfalls kugelförmig ausgebildet sind, d. h. die Form eines Kugelabschnittes aufweisen. Die Kugelköpfe 12, welche ebenfalls in Form eines Kugelabschnittes ausgebildet sind, und die Gelenkpfannen 16 sind bevorzugt so ausgebildet, dass sie im Wesentlichen spielfrei ineinander eingreifen können, wobei die Beweglichkeit der Klappenelemente 4 um die Schwenkachse S gewährleistet bleibt. So wird ein sehr schmaler bzw. dünner Gelenkspalt zwischen Kugelkopf 12 und Gelenkpfanne 16 sichergestellt, in welchem sich keine Ablagerungen bilden können. Die Kugelköpfe 12 sind mit den Vorsprüngen 14 und dem Ringkörper 2 vorzugsweise einstückig aus Metall, beispielsweise Titan, gebildet. Die Klappenelemente 4 können ebenfalls aus Metall, gegebenenfalls mit geeigneten Beschichtungen ausgebildet sein. Vorzugsweise sind die Klappenelemente 4 jedoch aus einer Kohlenstoffstruktur gebildet, welche zum einen eine hohe Verschleißfestigkeit aufweist und darüber hinaus eine Oberfläche aufweist, an welcher sich im Wesentlichen keine Ablagerungen bilden.

Wie in Figuren 1 und 2 gezeigt, sind in der vorgesehenen Strömungsrichtung des Blutes entlang der Längsachse X durch die Herzklappenprothese gesehen, die Gelenke 10 an der stromaufwärtigen ventrikulären Seite bzw. Oberfläche 18 der Klappenelemente 4 gelegen, welche der Herzkammer zugewandt sind. Die ventrikuläre Oberfläche 18 ist dabei diejenige Oberfläche der Klappenelemente 4, welche in Strömungsrichtung X von dem Blut angeströmt wird. Ferner sind die Gelenke 10 bzw. die Gelenkpfannen 16 der Gelenke 10 an den Klappenelementen 4 beabstandet von den Seitenkanten 6 und 8 im Zentralbereich der Klappenelemente 4 gelegen. Mit dieser Ausgestaltung wird erreicht, dass die Gelenke 10 vollständig in der Strömung liegen und so durchströmt werden, dass sich weder in den Gelenken 10 noch an den Vorsprüngen 14, welche ringseitig die Gelenke 10 tragen, Anlagerungen bilden können. Die Vorsprünge 14 weisen, wie in Fig. 4 zu erkennen ist, einen stromlinienförmigen bzw. strömungsoptimierten Querschnitt auf, welcher so gebildet ist, dass die Stege, in Strömungsrichtung X gesehen, sehr schmal ausgebildet sind und, in Strömungsrichtung X gesehen, sich an ihrer Rückseite im Wesentlichen keine Toträume ausbilden, in welchen sich Ablagerungen bilden könnten.

Die Vorsprünge 14 erstrecken sich in diesem Beispiel in einem spitzen Winkel zur Längsachse X, so dass die Vorsprünge 14 lediglich mit ihren Kugelköpfen 12 mit den Klappenelementen 4 in Kontakt kommen. Insbesondere liegen die Vorsprünge 14 in der geschlossenen Stellung, wie in Fig. 1 zu sehen ist, nicht an der Oberfläche der Klappenelemente 4 an. So wird verhindert, dass sich in diesem Bereich Ablagerungen bilden können.

### Bezugszeichenliste

- 2: - Ringkörper
- 4: - Klappenelemente
- 6, 8: - Seitenkanten der Klappelemente
- 10: - Gelenke
- 12: - Kugelkopf
- 14: - Vorsprung
- 16: - Gelenkpfanne
- 18: - ventrikuläre Oberfläche

- B: - Symmetrieachsen
- S: - Schwenkachsen
- X: - Längsachse und Strömungsrichtung des Blutes

## Patentansprüche

1. Herzklappenprothese mit einem Ringkörper (2) und mehreren mit dem Ringkörper (2) über Gelenke (10) beweglich verbundenen Klappenelementen (4), wobei die Gelenke (10) an den Klappenelementen (4) an deren ventrikulärer Oberfläche (18) angreifen, **dadurch gekennzeichnet, dass** die Gelenke (10) jeweils einen an dem Ringkörper (2) angebrachten Gelenkkopf (12) aufweisen, welcher in eine an der ventrikulären Oberfläche (18) des Klappenelementes (4) ausgebildete Gelenkpfanne (16) eingreift.

2. Herzklappenprothese nach Anspruch 1, **dadurch gekennzeichnet, dass** die Gelenke (10) an den Klappenelementen (4) jeweils beabstandet von deren Umfangskante (6, 8) angreifen.

3. Herzklappenprothese nach Anspruch 1 oder 2, **dadurch gekennzeichnet, dass** jedes Klappenelement (4) mit zwei Gelenken (10) an dem Ringkörper (2) befestigt ist, wobei die Gelenke (10) vorzugsweise symmetrisch an den Klappenelementen (4) angreifen.

4. Herzklappenprothese nach einem der vorangehenden Ansprüche, **dadurch gekennzeichnet, dass** die Gelenke (10) ringkörperseitig an Vorsprüngen (14) ausgebildet sind, welche sich in den Innenraum des Ringkörpers (2) hinein erstrecken.

5. Herzklappenprothese nach Anspruch 4, **dadurch gekennzeichnet, dass** die Vorsprünge (14) einen stromlinienförmigen Querschnitt aufweisen.

6. Herzklappenprothese nach einem der vorangehenden Ansprüche, **dadurch gekennzeichnet, dass** mindestens zwei, vorzugsweise drei Klappenelemente (4) in dem Ringkörper (2) angeordnet sind.

7. Herzklappenprothese nach einem der vorangehenden Ansprüche, **dadurch gekennzeichnet, dass** zumindest die Klappenelemente (4) aus Kohlenstoff gefertigt sind.

8. Herzklappenprothese nach einem der vorangehenden Ansprüche, **dadurch gekennzeichnet, dass** der Ringkörper (2) aus Metall gefertigt ist.

9. Herzklappenprothese nach einem der vorangehenden Ansprüche, **dadurch gekennzeichnet, dass** der Ringkörper (2) einstückig ausgebildet ist.

## Claims

1. A heart valve prosthesis with an annular body (2) and with several flap elements (4) which are movably connected to the annular body (2) via joints (10), wherein the joints (10) engage on the flap elements (4) at their ventricular surface (18), **characterised in that** the joints (10) each comprise a joint head (12) which is attached on the annular body (2) and which engages into a joint socket (16) which is formed on the ventricular surface (18) of the flap element (4).

2. A heart valve prosthesis according to claim 1, **characterised in that** the joints (10) engage on the flap elements (4) each in a manner distanced to their peripheral edge (6, 8).

3. A heart valve prosthesis according to claim 1 or 2, **characterised in that** each flap element (4) is fastened to the annular body (2) by two joints (10), wherein the joints (10) preferably engage on the flap elements body (4) in a symmetrical manner.

4. A heart valve prosthesis according to one of the preceding claims, **characterised in that** the joints (10) are formed on projections (14) at the annular body side, and these projections extend into the interior of the annular body (2).

5. A heart valve prosthesis according to claim 4, **characterised in that** the projections (14) have a streamline cross section.

6. A heart valve prosthesis according to one of the preceding claims, **characterised in that** at least two, preferably three flap elements (4) are arranged in the annular body (2).

7. A heart valve prosthesis according to one of the preceding claims, **characterised in that** at least the flap elements (4) are manufactured of carbon.

8. A heart valve prosthesis according to one of the preceding claims, **characterised in that** the annular body (2) is manufactured of metal.

9. A heart valve prosthesis according to one of the preceding claims, **characterised in that** the annular body (2) is designed as one piece.

## Revendications

1. Prothèse valvulaire cardiaque comprenant un corps annulaire (2) et plusieurs éléments de valve (4) reliés de façon mobile au corps annulaire (2) par le biais d'articulations (10), où les articulations (10) s'attachent aux éléments de valve (4) au niveau de leur surface ventriculaire (18), **caractérisée en ce que** les articulations (10) présentent chacune une tête d'articulation (12) placée au niveau du corps annulaire (2), qui s'insère dans une cavité d'articulation (16) formée au niveau de la surface ventriculaire (18) de l'élément de valve (4).

2. Prothèse valvulaire cardiaque selon la revendication 1, **caractérisée en ce que** les articulations (10) s'attachent au niveau des éléments de valve (4) en étant chacune éloignées de leur arête périphérique (6, 8).

3. Prothèse valvulaire cardiaque selon la revendication 1 ou 2, **caractérisée en ce que** chaque élément de valve (4) est fixé au corps annulaire (2) par deux articulations (10), les articulations (10) s'attachant de préférence de façon symétrique aux éléments de valve (4).

4. Prothèse valvulaire cardiaque selon l'une des revendications précédentes, **caractérisée en ce que** les articulations (10) sont formées côté corps annulaire au niveau de saillies (14) qui s'étendent jusque dans l'espace interne du corps annulaire (2).

5. Prothèse valvulaire cardiaque selon la revendication 4, **caractérisée en ce que** les saillies (14) présentent une section transversale de forme aérodynamique.

6. Prothèse valvulaire cardiaque selon l'une des revendications précédentes, **caractérisée en ce qu'**au moins deux, de préférence trois, éléments de valve (4) sont disposés dans le corps annulaire (2).

7. Prothèse valvulaire cardiaque selon l'une des revendications précédentes, **caractérisée en ce qu'**au moins des éléments de valve (4) sont fabriqués en carbone.

8. Prothèse valvulaire cardiaque selon l'une des revendications précédentes, **caractérisée en ce que** le corps annulaire (2) est fabriqué en métal.

9. Prothèse valvulaire cardiaque selon l'une des revendications précédentes, **caractérisée en ce que** le corps annulaire (2) est fabriqué d'une seule pièce.
